# EUROPEAN PATENT APPLICATION

(11) **EP 4 582 538 A1**
(43) Date of publication of application: **09.07.2025**
(21) Application number: 22956819.1
(22) Date of filing: 30.08.2022
(51) Int. Cl.: C12N 9/02, C12N 15/53, C12N 15/70, C12N 1/21, C12P 11/00, C12P 17/12, C12P 17/00

(54) **MONOOXYGENASE MUTANT AND USE THEREOF**

(71) Applicant: Asymchem Laboratories (Tianjin) Co., Ltd., Tianjin 300457 (CN)
(72) Inventor: HONG, Hao, Morrisville North Carolina 27560 (US); JAMES, Gage, Morrisville North Carolina 27560 (US); ZHANG, Na, Tianjin 300457 (CN); JIAO, Xuecheng, Tianjin 300457 (CN); MA, Yulei, Tianjin 300457 (CN); GENG, Yuhan, Tianjin 300457 (CN); MU, Huiyan, Tianjin 300457 (CN)
(74) Representative: Zacco GmbH
(86) International application number: PCT/CN2022/115947
(87) International publication number: WO 2024/044987

(57) **Abstract**

The present application provides a monooxygenase mutant and an application thereof. Herein, the monooxygenase mutant includes (a) a protein having an amino acid sequence shown in SEQ ID NO: 1; or (b) a protein that undergoes an amino acid mutation in at least one of the following sites: W60, Y65, D71, Y77, D163, V166, S178, T179, T199, G200, S201, R222 and the like of the amino acid sequence in (a) and has a monooxygenase function; and (c) a protein that has more than 80% of identity with the amino acid sequence defined in any one of (a) and (b) and has the monooxygenase function. The problem of low monooxygenase activity in prior art may be solved, and it is applied to the field of enzyme catalysis.

## Description

### Technical Field

The present invention relates to the field of enzyme catalysis, in particular to a monooxygenase mutant and an application thereof.

### Background

Chiral sulfoxides are a class of chiral organic sulfur compounds widely used as chiral auxiliaries or ligands for asymmetric catalysis, and bioactive molecules in the pharmaceutical industry. Many chiral sulfoxides contain one or more chiral centers, and there are significant differences in the pharmacological activity, metabolic processes, metabolic rates, and toxicity of different chiral drugs. Typically, one enantiomer is effective, while the other enantiomer is inefficient or ineffective. For example: the enantiomeric purity of omeprazole and lansoprazole, which are used as chiral sulfoxide gastrointestinal drugs, has a significant impact on the efficacy of this class of the drugs. Levorotatory omeprazole, also known as esomeprazole, has a better therapeutic effect than a dextroisomer; and dextrorotatory lansoprazole has a better therapeutic effect than a laevoisomer. Therefore, how to construct compounds containing chiral centers with high stereoselectivity is of great significance in pharmaceutical research and development.

The chiral sulfoxides may be synthesized by using both chemical method and biological method, and the chemical method includes chiral auxiliary induction, chiral resolution, and asymmetric catalysis and the like. However, there are some apparent drawbacks in the synthesis process, such as the use of harmful oxidants and organometallic reagents. In addition, the efficiency of high optical purity sulfoxide is not sufficient to achieve, and the downstream removal of isomers is often challenged. In contrast, the synthesis of the chiral sulfoxides using the biological method is increasingly favored by people due to its relatively mild reaction conditions, the "green" advantage without generating toxic wastes, and better enantioselectivity and non-enantioselectivity. However, wild-type monooxygenases commonly have problems such as narrow substrate range, low activity, poor stability, insufficient selectivity, and by-product sulfone generation. The monooxygenase may be modified by directed evolution methods, to improve various properties of the enzyme, thereby it may be applied in industrial production.

### Summary

A main purpose of the present invention is to provide a monooxygenase mutant and an application thereof, as to solve problems of low monooxygenase activity, insufficient product chirality, and many sulfone impurities in prior art.

In order to achieve the above purpose, according to an aspect of the present invention, a monooxygenase mutant is provided, and includes (a) a protein having an amino acid sequence shown in SEQ ID NO: 1; or (b) a protein that undergoes an amino acid mutation in at least one of the following sites: W60, Y65, D71, Y77, D163, V166, S178, T179, T199, G200, S201, R222, T223, H337, P338, K342, R343, S344, R365, I393, F395, D396, A397, L448, D505, S506, Y508, or R509 of the amino acid sequence in (a) and has a monooxygenase function; and (c) a protein that has more than 80% of identity with the amino acid sequence defined in any one of (a) and (b) and has the monooxygenase function.

Further, the amino acid mutation in (b) is independently selected from the following: D163V or D163A or D163E; V166S or V166Y or V166M or V166C or V166D or V166H or V166I or V166N or V166K or V166M or V166L or V166A or V166T or V166P or V166G or V166F; H337Y or H337M or H337K or H337P or H337F or H337A or H337L or H337E or H337D or H337R; P338A or P338M or P338L or P338Y; K342A or K342E or K342L or K342Y; R343A or R343Y or R343H or R343V; S344F or S344Y or S344L or S344M or S344K or S344A or S344T or S344N or S344R or S344D or S344E; R365A or R365G or R365D or R365T or R365Y; I393A or I393K or I393P or I393W or I393R or I393C or I393M or I393T or I393V or I393Y or I393G or I393L; F395S or F395Q or F395V; D396A or D396R or D396S or D396F or D396H or D396Q or D396K; A397V or A397L or A397I or A397M or A397R or A397H or A397W; herein, the letter before the number represents an original amino acid, and the letter after the number represents a mutated amino acid; and preferably, in (c), the protein has more than 85%, preferably more than 90%, more preferably more than 95%, and further preferably more than 99% of identity with the amino acid sequence defined in (a) or (b) and has the monooxygenase function.

Further, the mutation of the monooxygenase mutant includes any one of the following amino acid mutations: D163V, D163A, D163E, A397V, A397L, A397I, V166S, V166Y, V166M, I393A, I393K, I393P, D396A, D396R, D163A+A397L, D163A+A397V, D163A+ A397I, D163A+A397M, D163A+A397R, D163A+A397H, D163A+A397W, D163A+I393W, D163A+I393R, D163A+I393C, D163A+I393L, D163A+I393M, D163A+I393T, D163A+I393V, D163A+I393Y, D163A+I393G, D163A+V166C, D163A+V166D, D163A+V166H, D163A+V166I, D163A+V166N, D163A+V166K, D163A+V166M, D163A+V166L, D163A+H337Y, D163A+H337M, D163A+H337K, D163A+H337P, D163A+H337F, D163A+S344F, D163A+S344Y, D163A+S344L, D163A+S344M, D163A+S344K, D163A+S344A, D163A+S344T, D163A+A397L+I393R, D163A+A397L+I393C, D163A+A397L+I393L, D163A+A397L+I393M, D163A+A397L+I393T, D163A+A397L+I393V, D163A+A397L+V166I, D163A+A397L+V166C, D163A+A397L+V166H, D163A+A397L+V166A, D163A+A397L+V166Y, D163A+A397L+V166D, D163A+A397L+V166T, D163A+A397L+V166N, D163A+A397L+S344F, D163A+A397L+S344M, D163A+A397L+S344L, D163A+A397L+S344Y, D163A+A397L+S344K, D163A+A397L+S344N, D163A+A397L+S344R, D163A+A397L+I393L, D163A+A397L+I393L+V166C, D163A+A397L+I393L+V166H, D163A+A397L+I393L+V166D, D163A+A397L+I393L+V166I, D163A+A397L+I393L+V166N, D163A+A397L+I393L+V166P, D163A+A397L+I393L+V166G, D163A+A397L+I393L+V166F, D163A+A397L+I393L+V166L, D163A+A397L+I393L+H337Y, D163A+A397L+I393L+H337A, D163A+A397L+I393L+H337L, D163A+A397L+I393L+H337E, D163A+A397L+I393L+H337D, D163A+A397L+I393L+S344A, D163A+A397L+I393L+S344D, D163A+A397L+I393L+S344F, D163A+A397L+I393L+S344L, D163A+A397L+I393L+S344R, D163A+A397L+I393L+S344Y, D163A+A397L+I393L+V166I+H337Y, D163A+A397L+I393L+V166I+H337A, D163A+A397L+I393L+V1661+H337L, D163A+A397L+I393L+V1661+H337R, D163A+A397L+I393L+V166I+H337F, D163A+A397L+I393L+V166I+S344M, D163A+A397L+I393L+V166I+S344Y, D163A+A397L+I393L+V166I+S344L, D163A+A397L+I393L+V166I+S344F, D163A+A397L+I393L+V166I+S344A, D163A+A397L+I393L+V166I+S344E, D163A+A397L+I393L+V166I+S344T, D163A+A397L+I393L+V166I+S344Y+P338A, D163A+A397L+I393L+V166I+S344Y+P338M, D163A+A397L+I393L+V166I+S344Y+P338L, D163A+A397L+I393L+V166I+S344Y+P338Y, D163A+A397L+I393L+V166I+S344Y+K342A, D163A+A397L+I393L+V166I+S344Y+K342E, D163A+A397L+I393L+V166I+S344Y+K342L, D163A+A397L+I393L+V166I+S344Y+K342Y, D163A+A397L+I393L+V166I+S344Y+R343A, D163A+A397L+I393L+V166I+S344Y+R343A, D163A+A397L+I393L+V166I+S344Y+R343Y, D163A+A397L+I393L+V166I+S344Y+R343H, D163A+A397L+I393L+V166I+S344Y+R343V, D163A+A397L+I393L+V166I+S344Y+R365A, D163A+A397L+l393L+V166I+S344Y+R365G, D163A+A397L+I393L+V166I+S344Y+R365D, D163A+A397L+I393L+V166I+S344Y+R365T, D163A+A397L+I393L+V166I+S344Y+R365Y, D163A+A397L+I393L+V166I+S344Y+D396S, D163A+A397L+I393L+V166I+S344Y+D396F, D163A+A397L+I393L+V166I+S344Y+D396H, D163A+A397L+I393L+V166I+S344Y+F164A, D163A+A397L+I393L+V166I+S344Y+F164L, D163A+A397L+I393L+V166I+S344Y+F164M, D163A+A397L+I393L+V166I+S344Y+D165A, D163A+A397L+I393L+V166I+S344Y+D165M, D163A+A397L+I393L+V166I+S344Y+D165Y, D163A+A397L+I393L+V166I+S344Y+K162A, D163A+A397L+I393L+V166I+S344Y+K162S, D163A+A397L+I393L+V166I+S344Y+K162G, D163A+A397L+I393L+V166I+S344Y+G394A, D163A+A397L+I393L+V166I+S344Y+G394L, D163A+A397L+I393L+V166I+S344Y+G394T, D163A+A397L+I393L+V166I+S344Y+F395S, D163A+A397L+I393L+V166I+S344Y+F395Q, D163A+A397L+I393L+V166I+S344Y+F395V, D163A+A397L+I393L+V166I+S344Y+D396Q, D163A+A397L+I393L+V166I+S344Y+D396S, D163A+A397L+I393L+V166I+S344Y+D396F, D163A+A397L+I393L+V166I+S344Y+D396H, D163A+A397L+I393L+V166I+S344Y+D396K.

In order to achieve the above purpose, according to a second aspect of the present invention, a DNA molecule is provided, and the DNA molecule encodes the above monooxygenase mutant.

In order to achieve the above purpose, according to a third aspect of the present invention, a recombinant plasmid is provided, and the recombinant plasmid is linked to the above DNA molecule.

In order to achieve the above purpose, according to a fourth aspect of the present invention, a host cell is provided, and the above recombinant plasmid is transformed in the host cell.

Further, the host cell includes a prokaryotic cell; and preferably, the prokaryotic cell includes Escherichia coli.

In order to achieve the above purpose, according to a fifth aspect of the present invention, a preparation method for a chiral sulfoxide compound is provided, and the preparation method includes using the above monooxygenase mutant to perform an oxygenation reaction with a thioether substrate shown in Formula I and/or Formula II, to obtain the chiral sulfoxide compound,

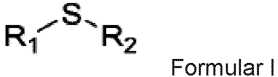

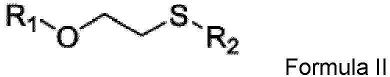

R₁ is selected from an alkyl, a cycloalkyl, an aryl or a heteroaryl, the number of C atoms of the alkyl is selected from 1-8, and the number of C atoms of the cycloalkyl, aryl or heteroaryl is selected from 5~10; R₂ is selected from an alkyl, a cycloalkyl, an aryl or a heteroaryl, the number of C atoms of the alkyl is selected from 1-8, and the number of C atoms of the cycloalkyl, aryl or heteroaryl is selected from 5~10; or R₁ and R₂ form a heterocyclyl, a carbocyclyl or a heteroaryl together with sulfur atoms, and the number of C atoms of the heterocyclyl, the carbocyclyl or the heteroaryl is selected from 5~10; heteroatoms in the heterocyclyl or the heteroaryl are each independently selected from at least one of nitrogen, oxygen or sulfur; and the aryl, the heteroaryl, the carbocyclyl or the heteroaryl is each independently unsubstituted or substituted, and when substituted, a substituent group is selected from at least one group of a halogen, an alkoxy or an alkyl.

Further, the thioether substrate is selected from

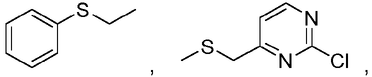

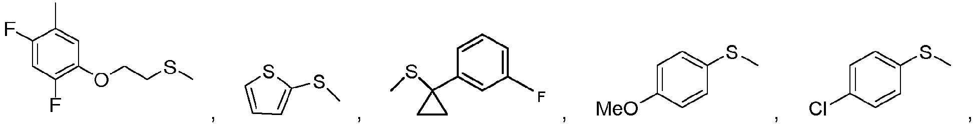

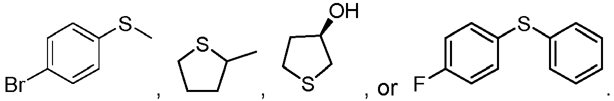

By applying technical schemes of the present invention, the monooxygenase mutant (SEQ ID NO: 1) derived from *Rhodococcus jostii RHA1* is used as a female parent, and protein engineering modifications such as a single point directed mutation, a saturation mutation, and a combination mutation are performed, to obtain the monooxygenase mutant that may generate high-purity optical pure chiral sulfoxide compounds with less sulfone impurities and high enzyme activity.

### Detailed Description of the Embodiments

It should be noted that, in the case without conflicting, embodiments in the present application and features in the embodiments may be combined with each other. The present invention is described in detail below in combination with the embodiments.

As mentioned in the background, the chemical synthesis of chiral sulfoxide has various deficiencies. The use of the biological method to synthesize the chiral sulfoxide is not only more "green" but also has the milder reaction, thus it becomes a preferred method for synthesizing this substance. However, wild-type monooxygenases used for biosynthesis mainly have the problem of low activity. In addition, there are also problems such as narrow substrate range, poor stability, insufficient selectivity, and by-product sulfone generation.

Therefore, in the present application, the inventor attempts to modify the monooxygenase by a method of directed evolution, thereby the various properties of the enzyme are improved, so that it may be used in a technical scheme for industrial production of the chiral sulfoxide. Therefore, a series of protection schemes are proposed in the present application.

In a first typical implementation mode of the present application, a monooxygenase mutant is provided, and includes (a) a protein having an amino acid sequence shown in SEQ ID NO: 1; or (b) a protein that undergoes an amino acid mutation in at least one of the following sites: W60, Y65, D71, Y77, D163, V166, S178, T179, T199, G200, S201, R222, T223, H337, P338, K342, R343, S344, R365, I393, F395, D396, A397, L448, D505, S506, Y508, or R509 of the amino acid sequence in (a) and has a monooxygenase function; and (c) a protein that has more than 80% of identity with the amino acid sequence defined in any one of (a) and (b) and has the monooxygenase function.

The above amino acid sequence shown in SEQ ID NO: 1 is an amino acid mutant derived from *Rhodococcus jostii RHA 1.* By computer simulation analysis of homologous modeling of the amino acid sequence and molecular docking results of a model structure and a thioether substrate, 28 amino acid residues are found, including: W60, Y65, D71, Y77, D163, V166, S178, T179, T199, G200, S201, R222, T223, H337, P338, K342, R343, S344, R365, I393, F395, D396, A397, L448, D505, S506, Y508, and R509. These amino acid sites are located near the active center and may affect the catalytic properties of the protein. By mutating the above amino acid sites, proteins with monooxygenase function or even enhanced monooxygenase function may be obtained. For the above proteins obtained, changes may be made in non-critical mutation sites and active sites, thus the protein that has more than 80% of identity with the above amino acid sequence and has the monooxygenase function may be obtained.

The sequence of SEQ ID NO: 1 is as follows:

MTTSMKAANPMNFPSTSDTGIVDVLGVGAGFSGLYLSHRLTTAGWTFAGFEAGPSVGGTWF
WNTYPGARCDVESIYYSYSFDEALQQEWTWSQRFAPQAEILSYINHVADRFDLRKHFTFNTRVVG
ATWNAAERLWEVQLDNGETRRGRYLISGAGGLSTPKDFDVPGLGNFTGLQVSTSRWNISLDDLA
GKRVAVIGTGSSGVQAIPLIAEVAEHVTVFQRTPNYVMPARNAELPLERVDSIKDDYPAIREECRHS
PGGIPDRPVTDKAFDVSAEERQRRYEAAYERSGFNGVGGEFADLLTDVEANRTASEFIHDKIREIV
EDPATAELLVPRYHPLGAKRSVFGTDYYETYNRPNVSLVSLRDEPIETMTANAIVTSKGTYEADAVV
LAIGFDAFTGPLYGLGLTGASGRKLQETWQDGIRTYLGMMTTDFPNFFMVAGPQSPALASNVVMTI
EQAVDWIADLIEHARDSGATLVEATPEGQNDWVDITEETVAQTLYATTDSWYRGSNVEGKPNTFM
GYVGGVGKYRRMCTEIAKRGYPGVRIDGETESPHLGPIHREIS.

In a preferred embodiment, the amino acid mutation in (b) is independently selected from the following: D163V or D163A or D163E; V166S or V166Y or V166M or V166C or V166D or V166H or V166I or V166N or V166K or V166M or V166L or V166A or V166T or V166P or V166G or V166F; H337Y or H337M or H337K or H337P or H337F or H337A or H337L or H337E or H337D or H337R; P338A or P338M or P338L or P338Y; K342A or K342E or K342L or K342Y; R343A or R343Y or R343H or R343V; S344F or S344Y or S344L or S344M or S344K or S344A or S344T or S344N or S344R or S344D or S344E; R365A or R365G or R365D or R365T or R365Y; I393A or I393K or I393P or I393W or I393R or I393C or I393M or I393T or I393V or I393Y or I393G or I393L; F395S or F395Q or F395V; D396A or D396R or D396S or D396F or D396H or D396Q or D396K; A397V or A397L or A397I or A397M or A397R or A397H or A397W; herein, the letter before the number represents an original amino acid, and the letter after the number represents a mutated amino acid; and preferably, in (c), the protein has more than 85%, preferably more than 90%, more preferably more than 95%, 96%, 97% or 98%, and further preferably more than 99% or 99.9% of identity with the amino acid sequence defined in (a) or (b) and has the monooxygenase function.

In the present application, the applicant continues to explore the above active sites and finds that the active sites mutate into different amino acids, correspondingly the protein activities are different, and specific mutations may enhance monooxygenase activity. By experimental exploration, it is found that the above specific mutations in the active sites may obtain proteins with enhanced activity. For amino acid mutation sites of the monooxygenase protein, flexible selection and combination may be made among the above mutations.

In a preferred embodiment, the mutation of the monooxygenase mutant includes any one of the following amino acid mutations: D163V, D163A, D163E, A397V, A397L, A397I, V166S, V166Y, V166M, I393A, I393K, I393P, D396A, D396R, D163A+A397L, D163A+A397V, D163A+ A397I, D163A+A397M, D163A+A397R, D163A+A397H, D163A+A397W, D163A+I393W, D163A+I393R, D163A+I393C, D163A+I393L, D163A+I393M, D163A+I393T, D163A+I393V, D163A+I393Y, D163A+I393G, D163A+V166C, D163A+V166D, D163A+V166H, D163A+V166I, D163A+V166N, D163A+V166K, D163A+V166M, D163A+V166L, D163A+H337Y, D163A+H337M, D163A+H337K, D163A+H337P, D163A+H337F, D163A+S344F, D163A+S344Y, D163A+S344L, D163A+S344M, D163A+S344K, D163A+S344A, D163A+S344T, D163A+A397L+I393R, D163A+A397L+I393C, D163A+A397L+I393L, D163A+A397L+I393M, D163A+A397L+I393T, D163A+A397L+I393V, D163A+A397L+V166I, D163A+A397L+V166C, D163A+A397L+V166H, D163A+A397L+V166A, D163A+A397L+V166Y, D163A+A397L+V166D, D163A+A397L+V166T, D163A+A397L+V166N, D163A+A397L+S344F, D163A+A397L+S344M, D163A+A397L+S344L, D163A+A397L+S344Y, D163A+A397L+S344K, D163A+A397L+S344N, D163A+A397L+S344R, D163A+A397L+I393L, D163A+A397L+I393L+V166C, D163A+A397L+I393L+V166H, D163A+A397L+I393L+V166D, D163A+A397L+I393L+V166I, D163A+A397L+I393L+V166N, D163A+A397L+I393L+V166P, D163A+A397L+I393L+V166G, D163A+A397L+I393L+V166F, D163A+A397L+I393L+V166L, D163A+A397L+I393L+H337Y, D163A+A397L+I393L+H337A, D163A+A397L+I393L+H337L D163A+A397L+I393L+H337E, D163A+A397L+I393L+H337D, D163A+A397L+I393L+S344A, D163A+A397L+I393L+S344D, D163A+A397L+I393L+S344F, D163A+A397L+I393L+S344L, D163A+A397L+I393L+S344R, D163A+A397L+I393L+S344Y, D163A+A397L+I393L+V166I+H337Y, D163A+A397L+I393L+V166I+H337A, D163A+A397L+I393L+V166I+H337L, D163A+A397L+I393L+V166I+H337R, D163A+A397L+I393L+V166I+H337F, D163A+A397L+I393L+V166I+S344M, D163A+A397L+I393L+V166I+S344Y, D163A+A397L+I393L+V166I+S344L, D163A+A397L+I393L+V166I+S344F, D163A+A397L+I393L+V166I+S344A, D163A+A397L+I393L+V166I+S344E, D163A+A397L+I393L+V166I+S344T, D163A+A397L+I393L+V166I+S344Y+P338A, D163A+A397L+I393L+V166I+S344Y+P338M, D163A+A397L+I393L+V166I+S344Y+P338L, D163A+A397L+I393L+V166I+S344Y+P338Y, D163A+A397L+I393L+V166I+S344Y+K342A, D163A+A397L+I393L+V166I+S344Y+K342E, D163A+A397L+I393L+V166I+S344Y+K342L, D163A+A397L+I393L+V166I+S344Y+K342Y, D163A+A397L+I393L+V166I+S344Y+R343A, D163A+A397L+I393L+V166I+S344Y+R343A, D163A+A397L+I393L+V166I+S344Y+R343Y, D163A+A397L+I393L+V166I+S344Y+R343H, D163A+A397L+I393L+V166I+S344Y+R343V, D163A+A397L+I393L+V166I+S344Y+R365A, D163A+A397L+I393L+V166I+S344Y+R365G, D163A+A397L+I393L+V166I+S344Y+R365D, D163A+A397L+I393L+V166I+S344Y+R365T, D163A+A397L+I393L+V166I+S344Y+R365Y, D163A+A397L+I393L+V166I+S344Y+D396S, D163A+A397L+I393L+V166I+S344Y+D396F, D163A+A397L+I393L+V166I+S344Y+D396H, D163A+A397L+I393L+V166I+S344Y+F164A, D163A+A397L+I393L+V166I+S344Y+F164L, D163A+A397L+I393L+V166I+S344Y+F164M, D163A+A397L+I393L+V166I+S344Y+D165A, D163A+A397L+I393L+V166I+S344Y+D165M, D163A+A397L+I393L+V166I+S344Y+D165Y, D163A+A397L+I393L+V166I+S344Y+K162A, D163A+A397L+I393L+V166I+S344Y+K162S, D163A+A397L+I393L+V166I+S344Y+K162G, D163A+A397L+I393L+V166I+S344Y+G394A, D163A+A397L+I393L+V166I+S344Y+G394L, D163A+A397L+I393L+V166I+S344Y+G394T, D163A+A397L+I393L+V166I+S344Y+F395S, D163A+A397L+I393L+V166i+S344Y+F395Q, D163A+A397L+I393L+V166I+S344Y+F395V, D163A+A397L+I393L+V166I+S344Y+D396Q, D163A+A397L+I393L+V166I+S344Y+D396S, D163A+A397L+I393L+V166I+S344Y+D396F, D163A+A397L+I393L+V166I+S344Y+D396H, D163A+A397L+I393L+V166I+S344Y+D396K.

The above amino acid mutations are all experimentally explored in the embodiments of the present application, and all have the monooxygenase activity. Compared to the female parent with the amino acid sequence shown in SEQ ID NO: 1, the monooxygenase mutant with high-purity optical pure chiral sulfoxide, high enzyme activity, and less sulfone impurities may be obtained.

In a second typical implementation mode of the present application, a DNA molecule is provided, and the DNA molecule encodes the above monooxygenase mutant.

In a third typical implementation mode of the present application, a recombinant plasmid is provided, and the recombinant plasmid is linked to the above DNA molecule.

The above DNA may encode the above monooxygenase mutant and may be linked onto the recombinant plasmid to form a circular DNA. The above DNA and recombinant plasmid may be transcribed and translated under the action of RNA polymerase, ribosome, tRNA and the like, to obtain the above monooxygenase mutant.

In a fourth typical implementation mode of the present application, a prokaryotic host cell is provided, and the above recombinant plasmid is transformed in the prokaryotic host cell.

By applying the above prokaryotic host cell, it is possible to replicate the recombinant plasmid and transcribe and translate the DNA molecule carried by the recombinant plasmid, and a large number of monooxygenase mutants are obtained. Broken protein purification, crude enzyme catalysis after breakage, or other modes are performed on the prokaryotic host cell with the method in prior art to obtain the monooxygenase mutant, and subsequent catalysis of sulfoxide compounds is performed. The host cell is a prokaryotic host cell of non-plant origin.

In a fifth typical implementation mode of the present application, a preparation method for a chiral sulfoxide compound is provided, and the preparation method includes using the above monooxygenase mutant to perform an oxygenation reaction with a thioether substrate shown in Formula I and/or Formula II, to obtain the chiral sulfoxide compound, R₁ is selected from an alkyl, a cycloalkyl, an aryl or a heteroaryl, the number of C atoms of the alkyl is selected from 1-8, and the number of C atoms of the cycloalkyl, aryl or heteroaryl is selected from 5~10; R₂ is selected from an alkyl, a cycloalkyl, an aryl or a heteroaryl, the number of C atoms of the alkyl is selected from 1-8, and the number of C atoms of the cycloalkyl, aryl or heteroaryl is selected from 5~10; or R₁ and R₂ form a heterocyclyl, a carbocyclyl or a heteroaryl together with sulfur atoms, and the number of C atoms of the heterocyclyl, the carbocyclyl or the heteroaryl is selected from 5~10; heteroatoms in the heterocyclyl or the heteroaryl are each independently selected from at least one of nitrogen, oxygen or sulfur; and the aryl, the heteroaryl, the carbocyclyl or the heteroaryl is each independently unsubstituted or substituted, and when substituted, a substituent group is selected from at least one group of a halogen, an alkoxy or an alkyl;

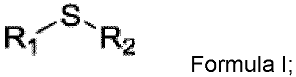

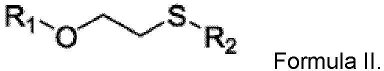

With the above preparation method and the above monooxygenase mutant, in the reaction of the provided thioether substrate shown in Formula I and/or Formula II, the chiral sulfoxide compound may be obtained by performing the oxygenation reaction on the thioether substrate shown in Formula I and/or Formula II. The above monooxygenase mutant may chirally catalyze the above thioether substrate to synthesize the chiral sulfoxide compound desired. The activity and selectivity of the monooxygenase are greatly improved, and the generation of sulfoxide impurities is reduced during the catalytic process, thus the production efficiency may be improved, the industrial production cost is reduced, and it is more suitable for industrial production.

In a preferred embodiment, the thioether substrate is selected from:

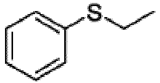

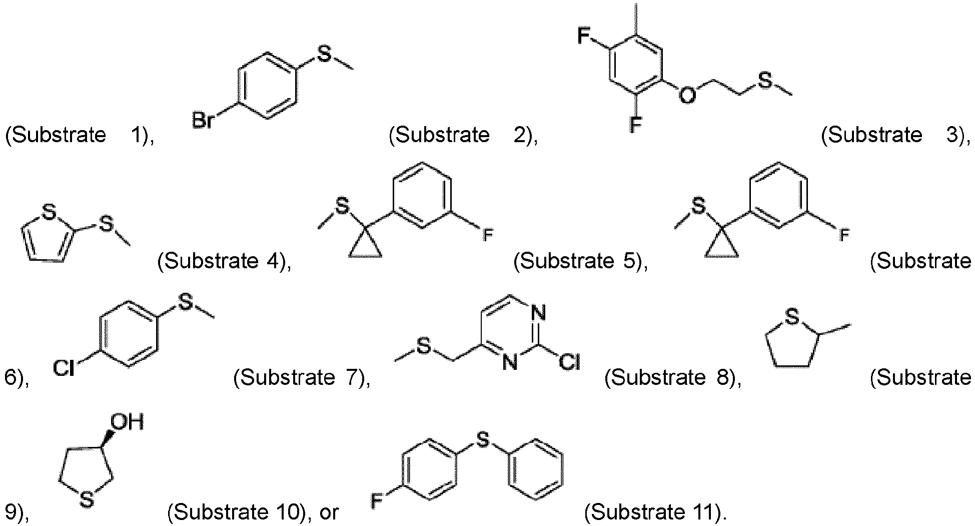

In the present application, by homologous modeling, the inventor performs molecular docking on the different thioether substrates according to the model structure, analyzes docking results, and selects 28 residues near the active center that may affect the catalytic properties of the protein, these residues include: W60, Y65, D71, Y77, D163, V166, S178, T179, T199, G200, S201, R222, T223, H337, P338, K342, R343, S344, R365, I393, F395, D396, A397, L448, D505, S506, Y508, and R509. Saturation mutation and site-specific mutation are performed on these residues.

Herein, the saturation mutation is a method of acquiring a mutant in which an amino acid in a target site is substituted by 19 other amino acids in a short period of time by modifying a coding gene of a target protein. This method is not only a powerful tool for protein directed modification, but also an important means of researching a protein structure-function relationship. The saturation mutation may often obtain more ideal evolutionary bodies than the single point mutation. For these problems that may not be solved by site-directed mutagenesis methods, it is precisely a unique point of saturation mutation methods. The mutant obtained from the saturation mutation is sequenced and identified, and its activity against different substrates and tolerance to high temperatures are tested separately.

Site-directed mutagenesis: refers to introduction of a desired change (usually a change that represents a favorable direction) into a target DNA fragment (may be a genome, or a plasmid) by methods such as a polymerase chain reaction (PCR), including addition of bases, deletion, point mutation and the like. The site-directed mutagenesis may rapidly and efficiently improve the characters and representation of a target protein expressed by DNA, and is a very useful means in genetic research work. The introduction method of the site-directed mutagenesis by using the whole plasmid PCR is simple and effective, and is a means which is often used at present. A principle thereof is: a pair of primers (forward and reverse) containing the mutation sites, and a template plasmid are annealed to "cyclic extension" with a polymerase, the so-called cyclic extension is that the polymerase extends the primer according to the template, it is returned and terminated at a 5'-end of the primer after a circle, and subjected to the cycle of repeated heating, annealing and extending. This reaction is different from rolling circle amplification, and may not form a plurality of tandem copies. After annealing, extension products of the forward and reverse primers are paired to become a nicked open-circle plasmid. A Dpn I enzyme-digested extension product, because the original template plasmid is derived from conventional Escherichia coli, is modified by dam methylation, is sensitive to Dpn I and is cut up, while the plasmid with a mutated sequence synthesized in vitro is not cut up because there is no methylation, and therefore it is successfully transformed in the subsequent transformation, to obtain a clone of a mutant plasmid.

The following high-throughput screening method is developed to screen for the mutant library.
1. Mutant culture: 300 µL of an LB culture medium is added to each well of a 96-well plate, and monoclonal clones from an agar plate are inoculated into the deep-well 96-well plate, and cultured overnight at 37°C and 200 rpm; bacterial solution cultured overnight is transferred to another 96-well plate of which each well is added with 800 µL of the LB culture medium by using Qpix, after being cultured at 37°C and 200 rpm for 5 h, and OD600 of the bacterial solution in the 96-well plate reaches 0.6-0.9, IPTG solution is added to the 96-well plate again by using Qpix, so that the final concentration of IPTG in the plate is 0.1 mM, and it is induced overnight at 25°C and 200 rpm for about 16 h; and it is centrifuged at 4000 rpm for 5 min, a supernatant is discarded, and it is reacted with whole cells.
2. 96-well plate high-throughput screening system: a reaction system: 0.6 mg of NADP+ and 0.5 wt of alcohol dehydrogenase, are added to the 96-well plate obtained in the first step, a phosphate buffer (PB, 0.2 M) with pH 8.0 is supplemented to a total volume of 300 µL and mixed uniformly. 3 mg of a substrate is dissolved in 20 µL of isopropanol to prepare substrate solution, the prepared substrate is added to the 96-well plate reaction system and mixed uniformly. It is reacted in a 20°C constant temperature shaker at 200 rpm for 16 h. After an overnight reaction, acetonitrile is added to the 96-well plate reaction system to terminate the reaction. It is centrifuged, a supernatant is absorbed into a shallow well plate for high performance liquid chromatography (HPLC) detection with a multi-channel pipette, and it is sealed with tin foil paper. High-throughput HPLC analysis is performed, to screen out mutants with improved properties.

The mutants screened from the mutant library are sequenced, the suitable mutants are selected according to sequencing results, and amplification reaction validation is performed. After multi-round evolution, the inventor obtains a series of monooxygenase mutants, these monooxygenase mutants significantly improve the activity and selectivity of various thioether substrates, and significantly reduce sulfone impurities. These mutants may be used for industrial production, and the catalytic efficiency is greatly improved.

The present application is further described in detail below in combination with specific embodiments, and these embodiments may not be understood as limiting the scope of protection claimed by the present application.

### Example 1

The above method was used, to perform site-specific mutagenesis on the basis of maternal SEQ ID NO: 1. Specific mutation sites were shown in Table 1, and the catalytic activity of the mutant was tested according to the following reaction conditions.

A reaction system included 100 mg of Substrate 1 or Substrate 2, 120 µL of isopropanol, 20 mg of NADP⁺, 5 mg of alcohol dehydrogenase dry powder, and 1 mL of monooxygenase crude enzyme solution (prepared from 100 mg of wet bacterial sludge), 0.2 M PB 8.0 was added to a total volume of 6 mL, and it was reacted in a 20°C constant temperature shaker at 200 rpm for 16 h. After the reaction was completed, 3 times of acetonitrile in volume was added to the system to terminate the reaction, it was centrifuged, a sample was taken, and sent for HPLC to analyze the conversion rate and enantiomeric excess (ee) value of the product. Results were shown in the table below.

The above wet bacterial sludge was obtained by centrifugation of the corresponding mutant *Escherichia coli* after fermentation, and the crude enzyme solution was obtained by adding pH 8.0 100 mM phosphate buffer to the wet bacterial sludge obtained, and then performing ultrasonic disruption.

Detection results were shown in Table 1.

**Table 1:**

| Mutation site | Conversion rate (%) | | ee value (%) | | Impurity sulfone (%) | |
|---|---|---|---|---|---|---|
| | Substrate 1 | Substrate 2 | Substrate 1 | Substrate 2 | Substrate 1 | Substrate 2 |
| Female parent | + | + | - | - | **** | **** |
| D163V | ++ | ++ | - | - | *** | *** |
| D163A | ++ | ++ | - | - | **** | **** |
| D163E | ++ | ++ | - | - | **** | **** |
| A397V | + | + | - | - | *** | *** |
| A397L | ++ | + | - | - | *** | *** |
| A397I | + | + | - | - | **** | *** |
| V166S | ++ | ++ | - | - | **** | *** |
| V166Y | ++ | + | - | - | **** | **** |
| V166M | + | + | -- | -- | **** | **** |
| I393A | ++ | + | -- | - | *** | **** |
| I393K | + | + | - | - | **** | *** |
| I393P | ++ | ++ | -- | - | **** | *** |
| D396A | + | + | -- | - | *** | *** |
| D396R | ++ | ++ | -- | - | **** | **** |

Note: In the above table, + represented that the conversion rate was <20%, ++ represented that the conversion rate was greater than or equal to 20% and less than 30%, and +++ represented that the conversion rate was greater than or equal to 30% and less than equal to 50%; - represented that the ee value was less than or equal to 90%, -- represented that the ee value was greater than 90% and less than or equal to 95%, --- represented that the ee value was greater than 95% and less than or equal to 98%, and ---- represented that the ee value was greater than 98% and less than or equal to 99.5%; and **** represented that the impurity sulfone content was greater than 5%, *** represented that the impurity sulfone content was less than or equal to 5% and greater than 2%, ** represented that the impurity sulfone content was less than or equal to 2% and greater than 0.5%, and * represented that the impurity sulfone content was less than or equal to 0.5%.

### Example 2

On the basis of Example 1, saturation mutation and combination mutation were performed, and activity screening was performed on the combination mutation under the same reaction conditions as Example 1. Results were shown in Table 2.

**Table 2:**

| Mutation site | Conversion rate (%) | | ee value (%) | | Impurity sulfone (%) | |
|---|---|---|---|---|---|---|
| | Substrate 1 | Substrate 2 | Substrate 1 | Substrate 2 | Substrate 1 | Substrate 2 |
| Female parent | + | + | - | - | **** | **** |
| D163A+A397L | +++ | +++ | -- | --- | *** | *** |
| D163A+A397V | +++ | +++ | --- | - | *** | **** |
| D163A+ A397I | +++ | +++ | - | - | *** | *** |
| D163A+A397M | +++ | +++ | -- | -- | *** | *** |
| D163A+A397R | +++ | +++ | --- | --- | *** | ** |
| D163A+A397H | + | + | - | -- | **** | *** |
| D163A+A397W | +++ | +++ | --- | --- | **** | *** |
| D163A+I393W | +++ | +++ | - | --- | **** | **** |
| D163A+I393R | +++ | +++ | -- | - | **** | *** |
| D163A+I393C | +++ | +++ | -- | - | *** | *** |
| D163A+I393L | +++ | +++ | - | - | **** | *** |
| D163A+I393M | +++ | ++ | -- | --- | **** | *** |
| D163A+I393T | +++ | +++ | -- | - | *** | *** |
| D163A+I393V | +++ | ++ | -- | --- | **** | **** |
| D163A+I393Y | +++ | +++ | - | --- | *** | **** |
| D163A+I393G | +++ | +++ | --- | --- | ** | *** |
| D163A+V166C | +++ | +++ | -- | - | **** | *** |
| D163A+V166D | +++ | +++ | - | --- | **** | **** |
| D163A+V166H | +++ | +++ | -- | -- | *** | ** |
| D163A+V166I | +++ | ++ | -- | -- | *** | ** |
| D163A+V166N | +++ | +++ | - | --- | **** | *** |
| D163A+V166K | +++ | +++ | -- | --- | **** | *** |
| D163A+V166M | +++ | +++ | --- | --- | *** | *** |
| D163A+V166L | +++ | ++ | -- | --- | **** | **** |
| D163A+H337Y | + | +++ | --- | --- | **** | *** |
| D163A+H337M | +++ | +++ | - | - | *** | *** |
| D163A+H337K | +++ | +++ | -- | - | **** | *** |
| D163A+H337P | +++ | +++ | - | - | *** | *** |
| D163A+H337F | +++ | ++ | - | - | *** | **** |
| D163A+S344F | +++ | +++ | - | - | *** | *** |
| D163A+S344Y | +++ | +++ | - | - | **** | *** |
| D163A+S344L | ++ | +++ | - | - | **** | *** |
| D163A+S344M | +++ | +++ | - | - | *** | **** |
| D163A+S344K | ++ | ++ | - | - | **** | *** |
| D163A+S344A | +++ | +++ | - | - | **** | *** |
| D163A+S344T | ++ | +++ | - | - | *** | *** |

Note: In the above table, + represented that the conversion rate was <20%, ++ represented that the conversion rate was greater than or equal to 20% and less than 30%, and +++ represented that the conversion rate was greater than or equal to 30% and less than equal to 50%; - represented that the ee value was less than or equal to 90%, -- represented that the ee value was greater than 90% and less than or equal to 95%, --- represented that the ee value was greater than 95% and less than or equal to 98%, and ---- represented that the ee value was greater than 98% and less than or equal to 99.5%; and **** represented that the impurity sulfone content was greater than 5%, *** represented that the impurity sulfone content was less than or equal to 5% and greater than 2%, ** represented that the impurity sulfone content was less than or equal to 2% and greater than 0.5%, and * represented that the impurity sulfone content was less than or equal to 0.5%.

### Example 3

On the basis of Example 2, saturation mutation and combination mutation were performed, and catalytic activity of the mutant was detected according to the following reaction conditions.

A reaction system included 100 mg of Substrate 1 or Substrate 2, 120 µL of isopropanol, 20 mg of NADP+, 5 mg of alcohol dehydrogenase dry powder, and 0.5 mL of monooxygenase crude enzyme solution (prepared from 50 mg of wet bacterial sludge), 0.2 M PB 8.0 was added to a total volume of 6 mL, and it was reacted in a 20°C constant temperature shaker at 200 rpm for 16 h. After the reaction was completed, 3 times of acetonitrile in volume was added to the system to terminate the reaction, it was centrifuged, a sample was taken, and sent for HPLC to analyze the conversion rate and ee value of the product. Results were shown in Table 3.

**Table 3:**

| Mutation site | Conversion rate (%) | | ee value (%) | | Impurity sulfone (%) | |
|---|---|---|---|---|---|---|
| | Substrate 1 | Substrate 2 | Substrate 1 | Substrate 2 | Substrate 1 | Substrate 2 |
| Female parent | + | + | - | - | **** | **** |
| D163A+A397L+I393R | ++ | +++ | --- | -- | *** | *** |
| D163A+A397L+I393C | +++ | ++ | --- | --- | *** | *** |
| D163A+A397L+I393L | +++ | +++ | --- | --- | ** | ** |
| D163A+A397L+I393M | +++ | +++ | --- | --- | *** | *** |
| D163A+A397L+I393T | ++ | +++ | --- | --- | *** | *** |
| D163A+A397L+I393V | +++ | +++ | --- | --- | *** | *** |
| D163A+A397L+V166I | +++ | ++ | --- | -- | *** | ** |
| D163A+A397L+V166C | +++ | +++ | --- | --- | *** | *** |
| D163A+A397L+V166H | +++ | +++ | --- | -- | ** | *** |
| D163A+A397L+V166A | +++ | +++ | --- | --- | *** | *** |
| D163A+A397L+V166Y | +++ | +++ | --- | ---- | *** | ** |
| D163A+A397L+V166D | ++ | +++ | --- | -- | *** | ** |
| D163A+A397L+V166T | +++ | ++ | -- | --- | *** | *** |
| D163A+A397L+V166N | +++ | +++ | --- | --- | *** | ** |
| D163A+A397L+S344F | +++ | +++ | --- | -- | *** | *** |
| D163A+A397L+S344M | +++ | +++ | --- | -- | ** | *** |
| D163A+A397L+S344L | ++ | +++ | --- | --- | ** | ** |
| D163A+A397L+S344Y | +++ | +++ | -- | -- | *** | *** |
| D163A+A397L+S344K | +++ | +++ | -- | --- | ** | ** |
| D163A+A397L+S344N | ++ | ++ | --- | -- | *** | *** |
| D163A+A397L+S344R | +++ | ++ | --- | --- | *** | *** |

Note: In the above table, + represented that the conversion rate was <20%, ++ represented that the conversion rate was greater than or equal to 20% and less than 30%, and +++ represented that the conversion rate was greater than or equal to 30% and less than equal to 50%; - represented that the ee value was less than or equal to 90%, -- represented that the ee value was greater than 90% and less than or equal to 95%, --- represented that the ee value was greater than 95% and less than or equal to 98%, and ---- represented that the ee value was greater than 98% and less than or equal to 99.5%; and **** represented that the impurity sulfone content was greater than 5%, *** represented that the impurity sulfone content was less than or equal to 5% and greater than 2%, ** represented that the impurity sulfone content was less than or equal to 2% and greater than 0.5%, and * represented that the impurity sulfone content was less than or equal to 0.5%.

### Example 4

On the basis of Example 3, saturation mutation and combination mutation were performed, and activity screening was performed on the combination mutation under the same reaction conditions as Example 3. Results were shown in Table 4.

**Table 4:**

| Mutation site | Conversion rate (%) | | ee value (%) | | Impurity sulfone (%) | |
|---|---|---|---|---|---|---|
| | Substrate 1 | Substrate 2 | Substrate 1 | Substrate 2 | Substrate 1 | Substrate 2 |
| Female parent | + | + | - | - | **** | **** |
| D163A+A397L+ I393L | +++ | +++ | - | --- | ** | ** |
| D163A+A397L+ I393L+V166C | ++++ | +++ | ---- | --- | ** | * |
| D163A+A397L+ I393L+V166H | +++ | ++++ | --- | --- | ** | ** |
| D163A+A397L+ I393L+V166D | ++++ | ++ | --- | ---- | ** | ** |
| D163A+A397L+ I393L+V166I | ++++ | ++++ | ---- | ---- | * | ** |
| D163A+A397L+ I393L+V166N | ++++ | +++ | --- | --- | ** | ** |
| D163A+A397L+ I393L+V166P | ++++ | +++ | --- | --- | ** | ** |
| D163A+A397L+ I393L+V166G | ++++ | ++++ | --- | --- | ** | * |
| D163A+A397L+ I393L+V166F | ++++ | ++++ | --- | ---- | ** | ** |
| D163A+A397L+ I393L+V166L | ++++ | ++ | ---- | --- | ** | ** |
| D163A+A397L+ I393L+H337Y | ++++ | +++ | --- | --- | ** | ** |
| D163A+A397L+ I393L+H337A | ++++ | ++++ | --- | --- | ** | ** |
| D163A+A397L+ I393L+H337L | ++++ | +++ | --- | ---- | ** | ** |
| D163A+A397L+ I393L+H337E | ++++ | +++ | ---- | --- | ** | ** |
| D163A+A397L+ I393L+H337D | ++++ | +++ | --- | --- | ** | ** |
| D163A+A397L+ I393L+S344A | ++++ | +++ | --- | --- | ** | ** |
| D163A+A397L+ I393L+S344D | ++++ | ++++ | ---- | --- | ** | ** |
| D163A+A397L+ I393L+S344F | ++++ | +++ | --- | --- | * | ** |
| D163A+A397L+ I393L+S344L | ++++ | ++++ | --- | ---- | ** | ** |
| D163A+A397L+ I393L+S344R | ++++ | ++++ | --- | --- | ** | ** |
| D163A+A397L+ I393L+S344Y | ++++ | +++ | ---- | --- | ** | ** |

Note: In the above table, + represented that the conversion rate was <20%, ++ represented that the conversion rate was greater than or equal to 20% and less than 30%, and +++ represented that the conversion rate was greater than or equal to 30% and less than equal to 50%; - represented that the ee value was less than or equal to 90%, -- represented that the ee value was greater than 90% and less than or equal to 95%, --- represented that the ee value was greater than 95% and less than or equal to 98%, and ---- represented that the ee value was greater than 98% and less than or equal to 99.5%; and **** represented that the impurity sulfone content was greater than 5%, *** represented that the impurity sulfone content was less than or equal to 5% and greater than 2%, ** represented that the impurity sulfone content was less than or equal to 2% and greater than 0.5%, and * represented that the impurity sulfone content was less than or equal to 0.5%.

### Example 5

On the basis of Example 4, saturation mutation and combination mutation were performed, and catalytic activity of the mutant was detected according to the following reaction conditions.

A reaction system included 100 mg of Substrate 1 or Substrate 2, 120 µL of isopropanol, 20 mg of NADP+, 5 mg of alcohol dehydrogenase dry powder, and 0.25 mL of monooxygenase crude enzyme solution (prepared from 25 mg of wet bacterial sludge), 0.2 M PB 8.0 was added to a total volume of 6 mL, and it was reacted in a 20°C constant temperature shaker at 200 rpm for 16 h. After the reaction was completed, 3 times of acetonitrile in volume was added to the system to terminate the reaction, it was centrifuged, a sample was taken, and sent for HPLC to analyze the conversion rate and ee value of the product. Results were shown in Table 5 and Table 6.

**Table 5:**

| Mutation site | Conversion rate (%) | | ee value (%) | |
|---|---|---|---|---|
| | Substrate 1 | Substrate 2 | Substrate 1 | Substrate 2 |
| Female parent | + | + | - | - |
| D163A+A397L+I393L+V166I+H337Y | ++++ | ++++ | ---- | --- |
| D163A+A397L+I393L+V166I+H337A | ++++ | +++++ | ---- | ---- |
| D163A+A397L+I393L+V166I+H337L | +++++ | +++++ | ---- | ---- |
| D163A+A397L+I393L+V166I+H337R | +++++ | ++++ | ---- | ---- |
| D163A+A397L+I393L+V166I+H337F | +++++ | ++++ | ---- | ---- |
| D163A+A397L+I393L+V166I+S344M | ++++ | +++++ | ---- | ---- |
| D163A+A397L+I393L+V166I+S344Y | ++++ | +++++ | ---- | ---- |
| D163A+A397L+I393L+V166I+S344L | ++++ | +++++ | ---- | ---- |
| D163A+A397L+I393L+V166I+S344F | +++++ | ++++ | ---- | ---- |
| D163A+A397L+I393L+V166I+S344A | +++++ | +++++ | ---- | ---- |
| D163A+A397L+I393L+V166I+S344E | ++++ | +++++ | ---- | ---- |
| D163A+A397L+I393L+V166I+S344T | ++++ | ++++ | ---- | ---- |
| D163A+A397L+I393L+V166I+S344Y+P338A | +++++ | ++++ | ---- | ---- |
| D163A+A397L+I393L+V166I+S344Y+P338M | +++++ | +++++ | ---- | ---- |
| D163A+A397L+I393L+V166I+S344Y+P338L | +++++ | ++++ | ---- | ---- |
| D163A+A397L+I393L+V166I+S344Y+P338Y | +++++ | +++++ | ---- | ---- |
| D163A+A397L+I393L+V166I+S344Y+K342A | +++++ | +++++ | ---- | ---- |
| D163A+A397L+I393L+V166I+S344Y+K342E | +++++ | ++++ | ---- | ---- |
| D163A+A397L+I393L+V166I+S344Y+K342L | +++++ | ++++ | ---- | ---- |
| D163A+A397L+I393L+V166I+S344Y+K342Y | +++++ | ++++ | ---- | ---- |
| D163A+A397L+I393L+V166I+S344Y+R343A | +++ | +++++ | ---- | ---- |
| D163A+A397L+I393L+V166I+S344Y+R343A | +++++ | +++++ | ---- | ---- |
| D163A+A397L+I393L+V166I+S344Y+R343Y | +++++ | +++++ | ---- | ---- |
| D163A+A397L+I393L+V166I+S344Y+R343H | ++++ | +++++ | ---- | ---- |
| D163A+A397L+I393L+V166I+S344Y+R343V | +++++ | ++++ | ---- | ---- |
| D163A+A397L+I393L+V166I+S344Y+R365A | +++++ | +++ | ---- | ---- |
| D163A+A397L+I393L+V166I+S344Y+R365G | +++++ | ++++ | ---- | ---- |
| D163A+A397L+I393L+V166I+S344Y+R365D | +++++ | +++++ | ---- | ---- |
| D163A+A397L+I393L+V166I+S344Y+R365T | +++++ | ++++ | ---- | ---- |
| D163A+A397L+I393L+V166I+S344Y+R365Y | +++++ | +++++ | ---- | ---- |
| D163A+A397L+I393L+V166I+S344Y+D396S | +++++ | +++++ | --- | ---- |
| D163A+A397L+I393L+V166I+S344Y+D396F | +++++ | ++++ | ---- | ---- |
| D163A+A397L+I393L+V166I+S344Y+D396H | +++++ | +++++ | ---- | ---- |
| D163A+A397L+I393L+V166I+S344Y+F164A | ++++ | +++++ | ---- | ---- |
| D163A+A397L+I393L+V166I+S344Y+F164L | +++++ | ++++ | ---- | ---- |
| D163A+A397L+I393L+V166I+S344Y+F164M | +++++ | +++ | ---- | --- |
| D163A+A397L+I393L+V166I+S344Y+D165A | +++++ | ++++ | ---- | ---- |
| D163A+A397L+I393L+V166I+S344Y+D165M | +++++ | +++++ | ---- | ---- |
| D163A+A397L+I393L+V166I+S344Y+D165Y | +++++ | +++++ | ---- | ---- |
| D163A+A397L+I393L+V166I+S344Y+K162A | +++++ | +++++ | ---- | ---- |
| D163A+A397L+I393L+V166I+S344Y+K162S | +++++ | +++++ | ---- | ---- |
| D163A+A397L+I393L+V166I+S344Y+K162G | ++++ | +++++ | ---- | ---- |
| D163A+A397L+I393L+V166I+S344Y+G394A | +++++ | ++ | ---- | ---- |
| D163A+A397L+I393L+V166I+S344Y+G394L | +++++ | ++++ | ---- | ---- |
| D163A+A397L+I393L+V166I+S344Y+G394T | +++++ | +++++ | ---- | ---- |
| D163A+A397L+I393L+V166I+S344Y+F395S | +++++ | +++++ | ---- | ---- |
| D163A+A397L+I393L+V166I+S344Y+F395Q | +++++ | +++++ | ---- | ---- |
| D163A+A397L+I393L+V166I+S344Y+F395V | +++++ | +++++ | ---- | ---- |
| D163A+A397L+I393L+V166I+S344Y+D396Q | +++++ | +++++ | ---- | ---- |
| D163A+A397L+I393L+V166I+S344Y+D396S | ++++ | +++++ | ---- | ---- |
| D163A+A397L+I393L+V166I+S344Y+D396F | +++++ | +++++ | ---- | ---- |
| D163A+A397L+I393L+V166I+S344Y+D396H | ++++ | +++++ | ---- | ---- |
| D163A+A397L+I393L+V166I+S344Y+D396K | +++++ | +++++ | ---- | ---- |

**Table 6:**

| Mutation site | Impurity sulfone (%) | |
|---|---|---|
| | Substrate 1 | Substrate 2 |
| Female parent | **** | **** |
| D163A+A397L+I393L+V166I+H337Y | ** | * |
| D163A+A397L+I393L+V166I+H337A | ** | ** |
| D163A+A397L+I393L+V166I+H337L | ** | * |
| D163A+A397L+I393L+V166I+H337R | ** | ** |
| D163A+A397L+I393L+V166I+H337F | ** | * |
| D163A+A397L+I393L+V166I+S344M | ** | ** |
| D163A+A397L+I393L+V166I+S344Y | * | * |
| D163A+A397L+I393L+V166I+S344L | ** | * |
| D163A+A397L+I393L+V166I+S344F | ** | * |
| D163A+A397L+I393L+V166I+S344A | ** | ** |
| D163A+A397L+I393L+V166I+S344E | ** | * |
| D163A+A397L+I393L+V166I+S344T | ** | * |
| D163A+A397L+I393L+V166I+S344Y+P338A | ** | ** |
| D163A+A397L+I393L+V166I+S344Y+P338M | ** | ** |
| D163A+A397L+I393L+V166I+S344Y+P338L | ** | ** |
| D163A+A397L+I393L+V166I+S344Y+P338Y | ** | ** |
| D163A+A397L+I393L+V166I+S344Y+K342A | * | ** |
| D163A+A397L+I393L+V166I+S344Y+K342E | ** | ** |
| D163A+A397L+I393L+V166I+S344Y+K342L | ** | * |
| D163A+A397L+I393L+V166I+S344Y+K342Y | ** | ** |
| D163A+A397L+I393L+V166I+S344Y+R343A | ** | ** |
| D163A+A397L+I393L+V166I+S344Y+R343A | ** | * |
| D163A+A397L+I393L+V166I+S344Y+R343Y | ** | ** |
| D163A+A397L+I393L+V166I+S344Y+R343H | ** | ** |
| D163A+A397L+I393L+V166I+S344Y+R343V | * | ** |
| D163A+A397L+I393L+V166I+S344Y+R365A | ** | * |
| D163A+A397L+I393L+V166I+S344Y+R365G | * | ** |
| D163A+A397L+I393L+V166I+S344Y+R365D | ** | * |
| D163A+A397L+I393L+V166I+S344Y+R365T | ** | * |
| D163A+A397L+I393L+V166I+S344Y+R365Y | ** | * |
| D163A+A397L+I393L+V166I+S344Y+D396S | ** | * |
| D163A+A397L+I393L+V166I+S344Y+D396F | * | ** |
| D163A+A397L+I393L+V166I+S344Y+D396H | ** | * |
| D163A+A397L+I393L+V166I+S344Y+F164A | ** | ** |
| D163A+A397L+I393L+V166I+S344Y+F164L | ** | * |
| D163A+A397L+I393L+V166I+S344Y+F164M | ** | * |
| D163A+A397L+I393L+V166I+S344Y+D165A | ** | * |
| D163A+A397L+I393L+V166I+S344Y+D165M | ** | * |
| D163A+A397L+I393L+V166I+S344Y+D165Y | ** | * |
| D163A+A397L+I393L+V166I+S344Y+K162A | ** | * |
| D163A+A397L+I393L+V166I+S344Y+K162S | ** | ** |
| D163A+A397L+I393L+V166I+S344Y+K162G | ** | * |
| D163A+A397L+I393L+V166I+S344Y+G394A | ** | * |
| D163A+A397L+I393L+V166I+S344Y+G394L | ** | * |
| D163A+A397L+I393L+V166I+S344Y+G394T | ** | ** |
| D163A+A397L+I393L+V166I+S344Y+F395S | ** | * |
| D163A+A397L+I393L+V166I+S344Y+F395Q | ** | * |
| D163A+A397L+I393L+V166I+S344Y+F395V | ** | * |
| D163A+A397L+I393L+V166I+S344Y+D396Q | * | ** |
| D163A+A397L+I393L+V166I+S344Y+D396S | ** | * |
| D163A+A397L+I393L+V166I+S344Y+D396F | * | * |
| D163A+A397L+I393L+V166I+S344Y+D396H | * | ** |
| D163A+A397L+I393L+V166I+S344Y+D396K | ** | ** |

Note: In the above table, + represented that the conversion rate was <20%, ++ represented that the conversion rate was greater than or equal to 20% and less than 30%, and +++ represented that the conversion rate was greater than or equal to 30% and less than equal to 50%; - represented that the ee value was less than or equal to 90%, -- represented that the ee value was greater than 90% and less than or equal to 95%, --- represented that the ee value was greater than 95% and less than or equal to 98%, and ---- represented that the ee value was greater than 98% and less than or equal to 99.5%; and **** represented that the impurity sulfone content was greater than 5%, *** represented that the impurity sulfone content was less than or equal to 5% and greater than 2%, ** represented that the impurity sulfone content was less than or equal to 2% and greater than 0.5%, and * represented that the impurity sulfone content was less than or equal to 0.5%.

### Example 6

56 mL of 200 mmol/L phosphate buffer solution, 1.2 mL of isopropanol, 200 mg of NADP+, and 50 mg of alcohol dehydrogenase dry powder were added to a 250 mL four-necked flask at a room temperature, and pH was adjusted to 8.0. 1 g of 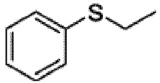 (Substrate 1) was added, and stirred uniformly, and then 2.5 mL (0.1 g wet bacterial sludge/mL) of enzyme solution of a monooxygenase mutant (D163A+A397L+I393L+V166I+S344Y) mutated on the basis of SEQ ID NO: 1 was added, and pH was adjusted to 8.0. The temperature was controlled to 20°C, and it was stirred and reacted overnight. After the reaction was completed, the system was acid-adjusted to pH=2-3, and the protein was denatured. A filtered filtrate was extracted with 60 mL of dichloromethane for 2 times. After a combined organic phase was dried with anhydrous magnesium sulfate, it was concentrated until there was no fraction under the conditions of T<35°C and P≤-0.08 Mpa. A target product 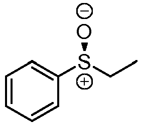 was obtained.

After being detected by HPLC, the purity was >99%, the ee value was >99%, and the yield was 82%.

### Example 7

56 mL of 200 mmol/L phosphate buffer solution, 1.2 mL of isopropanol, 200 mg of NADP+, and 50 mg of alcohol dehydrogenase dry powder were added to a 250 mL four-necked flask at a room temperature, and pH was adjusted to 8.0. 1 g of 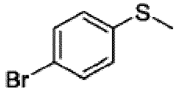 (Substrate 2) was added, and stirred uniformly, and then 2.5 mL (0.1 g wet bacterial sludge/mL) of enzyme solution of a monooxygenase mutant (D163A+A397L+I393L+V166I+S344Y) mutated on the basis of SEQ ID NO: 1 was added, and pH was adjusted to 8.0. The temperature was controlled to 20°C, and it was stirred and reacted overnight. After the reaction was completed, the system was acid-adjusted to pH=2-3, and the protein was denatured. A filtered filtrate was extracted with 60 mL of dichloromethane for 2 times. After a combined organic phase was dried with anhydrous magnesium sulfate, it was concentrated until there was no fraction under the conditions of T<35°C and P≤-0.08 Mpa. A target product 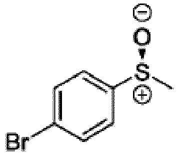 was obtained.

After being detected by HPLC, the purity was >98%, the ee value was >99%, and the yield was 84%.

### Example 8

56 mL of 200 mmol/L phosphate buffer solution, 1.2 mL of isopropanol, 200 mg of NADP+, and 50 mg of alcohol dehydrogenase dry powder were added to a 250 mL four-necked flask at a room temperature, and pH was adjusted to 8.0. 1 g of 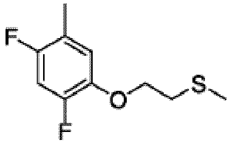 (Substrate 3) was added, and stirred uniformly, and then 2.5 mL (0.1 g wet bacterial sludge/mL) of enzyme solution of a monooxygenase mutant (D163A+A397L+I393L+V166I+S344Y) mutated on the basis of SEQ ID NO: 1 was added, and pH was adjusted to 8.0. The temperature was controlled to 20°C, and it was stirred and reacted overnight. After the reaction was completed, the system was acid-adjusted to pH=2-3, and the protein was denatured. A filtered filtrate was extracted with 60 mL of dichloromethane for 2 times. After a combined organic phase was dried with anhydrous magnesium sulfate, it was concentrated until there was no fraction under the conditions of T<35°C and P≤-0.08 Mpa. A target product 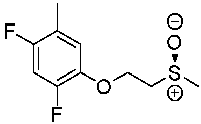 was obtained.

After being detected by HPLC, the purity was >99%, the ee value was >99%, and the yield was 87%.

### Example 9

Enzyme solution of a monooxygenase mutant (D163A+A397L+I393L+V166I+S344Y) mutated on the basis of SEQ ID NO: 1 was used, and a catalytic reaction was performed on Substrates 4-11 by referring to the catalytic synthesis steps of Examples 6 to 8. Results were shown in Table 7:

**Table 7:**

| Substrate | Purity | ee value | Yield |
|---|---|---|---|
| 4 | >99% | >99% | 84% |
| 5 | >99% | >99% | 81% |
| 6 | >98% | >99% | 81% |
| 7 | >98% | >99% | 84% |
| 8 | >99% | >99% | 86% |
| 9 | >99% | >99% | 84% |
| 10 | >99% | >99% | 84% |
| 11 | >99% | >99% | 82% |

From the above description, it may be seen that the above embodiments of the present invention achieve the following technical effects: based on the obtained monooxygenase female parent with excellent activity, the present application finds a plurality of sites that may improve the activity of the monooxygenase by exploring the active sites of the monooxygenase. By exploring the combination of one or more sites, a plurality of monooxygenase mutants with excellent performance is obtained. The above monooxygenase mutants may obtain high-purity optical pure chiral sulfoxide compounds, and have the advantages of high enzyme activity and less sulfone impurities generated, which may meet the needs of process production.

The above are only preferred embodiments of the present invention and are not intended to limit the present invention. For those skilled in the art, the present invention may have various modifications and variations. Any modifications, equivalent replacements, improvements and the like made within the spirit and principles of the present invention shall be contained within the scope of protection of the present invention.

## Claims

1. A monooxygenase mutant, comprising:
(a) a protein having an amino acid sequence as shown in SEQ ID NO:1; or
(b) a protein with a monooxygenase function that is undergone an amino acid mutation at one or more of the following sites: W60, Y65, D71, Y77, D163, V166, S178, T179, T199, G200, S201, R222, T223, H337, P338, K342, R343, S344, R365, I393, F395, D396, A397, L448, D505, S506, Y508, or R509 of the amino acid sequence in (a);
(c) a protein that has the monooxygenase function and is of more than 80% identity with the amino acid sequence defined in (a) or (b).

2. The monooxygenase mutant according to claim 1, wherein the amino acid mutation of (b) is independently selected from the following:
D163V, D163A, or D163E;
V166S, V166Y, V166M, V166C, V166D, V166H, V166I, V166N, V166K, V166M, V166L, V166A, V166T, V166P, V166G, or V166F;
H337Y, H337M, H337K, H337P, H337F, H337A, H337L, H337E, H337D, or H337R;
P338A, P338M, P338L, or P338Y;
K342A, K342E, K342L, or K342Y;
R343A, R343Y, R343H, or R343V;
S344F, S344Y, S344L, S344M, S344K, S344A, S344T, S344N, S344R, S344D, or S344E;
R365A, R365G, R365D, R365T, or R365Y;
I393A, I393K, I393P, I393W, I393R, I393C, I393M, I393T, I393V, I393Y, I393G, or I393L;
F395S, F395Q, or F395V;
D396A, D396R, D396S, D396F, D396H, D396Q, or D396K;
A397V, A397L, A397I, A397M, A397R, A397H, or A397W, wherein
a letter before a number represents an original amino acid, and a letter after the number represents a mutated amino acid; and
preferably, in (c), the protein with the monooxygenase function and is of more than 85%, preferably, more than 90%, more preferably, more than 95%, and further preferably, more than 99% identity with the amino acid sequence defined in (a) or (b).

3. The monooxygenase mutant according to claim 2, wherein the monooxygenase mutant comprises any of the following amino acid mutation:
| |
|---|
| D163V |
| D163A |
| D163E |
| A397V |
| A397L |
| A397I |
| V166S |
| V166Y |
| V166M |
| I393A |
| I393K |
| I393P |
| D396A |
| D396R |
| D163A+A397L |
| D163A+A397V |
| D163A+ A397I |
| D163A+A397M |
| D163A+A397R |
| D163A+A397H |
| D163A+A397W |
| D163A+I393W |
| D163A+I393R |
| D163A+I393C |
| D163A+I393L |
| D163A+I393M |
| D163A+I393T |
| D163A+I393V |
| D163A+I393Y |
| D163A+I393G |
| D163A+V166C |
| D163A+V166D |
| D163A+V166H |
| D163A+V166I |
| D163A+V166N |
| D163A+V166K |
| D163A+V166M |
| D163A+V166L |
| D163A+H337Y |
| D163A+H337M |
| D163A+H337K |
| D163A+H337P |
| D163A+H337F |
| D163A+S344F |
| D163A+S344Y |
| D163A+S344L |
| D163A+S344M |
| D163A+S344K |
| D163A+S344A |
| D163A+S344T |
| D163A+A397L+I393R |
| D163A+A397L+I393C |
| D163A+A397L+I393L |
| D163A+A397L+I393M |
| D163A+A397L+I393T |
| D163A+A397L+I393V |
| D163A+A397L+V166I |
| D163A+A397L+V166C |
| D163A+A397L+V166H |
| D163A+A397L+V166A |
| D163A+A397L+V166Y |
| D163A+A397L+V166D |
| D163A+A397L+V166T |
| D163A+A397L+V166N |
| D163A+A397L+S344F |
| D163A+A397L+S344M |
| D163A+A397L+S344L |
| D163A+A397L+S344Y |
| D163A+A397L+S344K |
| D163A+A397L+S344N |
| D163A+A397L+S344R |
| D163A+A397L+I393L |
| D163A+A397L+I393L+V166C |
| D163A+A397L+I393L+V166H |
| D163A+A397L+I393L+V166D |
| D163A+A397L+I393L+V166I |
| D163A+A397L+I393L+V166N |
| D163A+A397L+I393L+V166P |
| D163A+A397L+I393L+V166G |
| D163A+A397L+I393L+V166F |
| D163A+A397L+I393L+V166L |
| D163A+A397L+I393L+H337Y |
| D163A+A397L+I393L+H337A |
| D163A+A397L+I393L+H337L |
| D163A+A397L+I393L+H337E |
| D163A+A397L+I393L+H337D |
| D163A+A397L+I393L+S344A |
| D163A+A397L+I393L+S344D |
| D163A+A397L+I393L+S344F |
| D163A+A397L+I393L+S344L |
| D163A+A397L+I393L+S344R |
| D163A+A397L+I393L+5344Y |
| D163A+A397L+I393L+V166I+H337Y |
| D163A+A397L+I393L+V166I+H337A |
| D163A+A397L+I393L+V166I+H337L |
| D163A+A397L+I393L+V166I+H337R |
| D163A+A397L+I393L+V166I+H337F |
| D163A+A397L+I393L+V166I+S344M |
| D163A+A397L+I393L+V166I+S344Y |
| D163A+A397L+I393L+V166I+S344L |
| D163A+A397L+I393L+V166I+S344F |
| D163A+A397L+I393L+V166I+S344A |
| D163A+A397L+I393L+V166I+S344E |
| D163A+A397L+I393L+V166I+S344T |
| D163A+A397L+I393L+V166I+S344Y+P338A |
| D163A+A397L+I393L+V166I+S344Y+P338M |
| D163A+A397L+I393L+V166I+S344Y+P338L |
| D163A+A397L+I393L+V166I+S344Y+P338Y |
| D163A+A397L+I393L+V166I+S344Y+K342A |
| D163A+A397L+I393L+V166I+S344Y+K342E |
| D163A+A397L+I393L+V166I+S344Y+K342L |
| D163A+A397L+I393L+V166I+S344Y+K342Y |
| D163A+A397L+I393L+V166I+S344Y+R343A |
| D163A+A397L+I393L+V166I+S344Y+R343A |
| D163A+A397L+I393L+V166I+S344Y+R343Y |
| D163A+A397L+I393L+V166I+S344Y+R343H |
| D163A+A397L+I393L+V166I+S344Y+R343V |
| D163A+A397L+I393L+V166I+S344Y+R365A |
| D163A+A397L+I393L+V166I+S344Y+R365G |
| D163A+A397L+I393L+V166I+S344Y+R365D |
| D163A+A397L+I393L+V166I+S344Y+R365T |
| D163A+A397L+I393L+V166I+S344Y+R365Y |
| D163A+A397L+I393L+V166I+S344Y+D396S |
| D163A+A397L+I393L+V166I+S344Y+D396F |
| D163A+A397L+I393L+V166I+S344Y+D396H |
| D163A+A397L+I393L+V166I+S344Y+F164A |
| D163A+A397L+I393L+V166I+S344Y+F164L |
| D163A+A397L+I393L+V166I+S344Y+F164M |
| D163A+A397L+I393L+V166I+S344Y+D165A |
| D163A+A397L+I393L+V166I+S344Y+D165M |
| D163A+A397L+I393L+V166I+S344Y+D165Y |
| D163A+A397L+I393L+V166I+S344Y+K162A |
| D163A+A397L+I393L+V166I+S344Y+K162S |
| D163A+A397L+I393L+V166I+S344Y+K162G |
| D163A+A397L+I393L+V166I+S344Y+G394A |
| D163A+A397L+I393L+V166I+S344Y+G394L |
| D163A+A397L+I393L+V166I+S344Y+G394T |
| D163A+A397L+I393L+V166I+S344Y+F395S |
| D163A+A397L+I393L+V166I+S344Y+F395Q |
| D163A+A397L+I393L+V166I+S344Y+F395V |
| D163A+A397L+I393L+V166I+S344Y+D396Q |
| D163A+A397L+I393L+V166I+S344Y+D396S |
| D163A+A397L+I393L+V166I+S344Y+D396F |
| D163A+A397L+I393L+V166I+S344Y+D396H |
| D163A+A397L+I393L+V166I+S344Y+D396K |

4. A DNA molecule, encoding the monooxygenase mutant according to any of claims 1 to 3.

5. A recombinant plasmid, connected with the DNA molecule according to claim 4.

6. A host cell, wherein the recombinant plasmid according to claim 5 is transformed in the prokaryotic host cell.

7. A method for preparing a chiral sulfoxide compound, comprising performing an oxygenation reaction with the monooxygenase mutant according to any of claims 1 to 3 and a thioether substrate shown in a formula I or a formula II, to obtain the chiral sulfoxide compound, 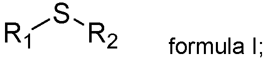 and 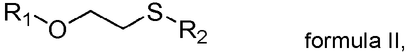
wherein R₁ is selected from a alkyl, a cycloalkyl, a aryl or a heteroaryl, the number of C atoms of the alkyl is selected from 1 to 8, and the number of C atoms of the cycloalkyl, aryl or heteroaryl is selected from 5 to 10;
R₂ is selected from a alkyl, a cycloalkyl, a aryl or a heteroaryl, the number of C atoms of the alkyl is selected from 1 to 8, and the number of C atoms of the cycloalkyl, aryl or heteroaryl is selected from 5 to 10; or
R₁ and R₂ form a heterocyclyl, a carbocyclyl or a heteroaryl together with sulfur atoms, and the number of C atoms of the heterocyclyl, the carbocyclyl or the heteroaryl is selected from 5 to 10;
the heteroatom in the heterocyclyl or the heteroaryl is each independently selected from at least one of nitrogen, oxygen or sulfur; and
the aryl, the heteroaryl, the carbocyclyl or the heteroaryl is each independently unsubstituted or substituted, and when substituted, a substituent group is selected from at least one group of a halogen, a alkoxy or a alkyl.

8. The preparation method according to claim 7, wherein the thioether substrate is selected from: 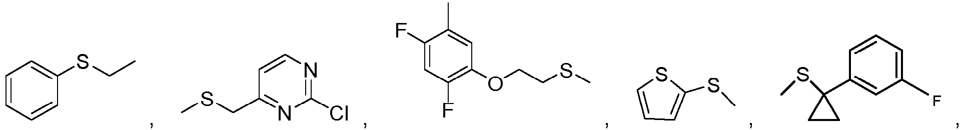 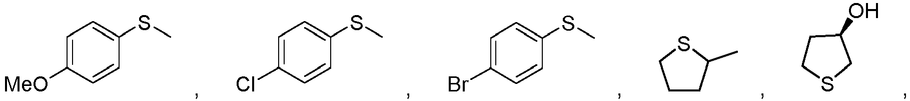 or
